# EUROPEAN PATENT APPLICATION

(11) **EP 4 430 966 A1**
(43) Date of publication of application: **18.09.2024**
(21) Application number: 22893357.8
(22) Date of filing: 05.10.2022
(51) Int. Cl.: A24F 40/10, A61K 31/465, A61P 25/34

(54) **SMART SYSTEM FOR COMBATING NICOTINE DEPENDENCE**

(30) Priority: 09.11.2021 RU 2021132504
(71) Applicant: Alfabet Labs DMCC, Dubai (AE)
(72) Inventor: NAZAROV, Marlen Petrovich, Moskovskaia oblast, g. Odincovo, 143005 (RU)
(74) Representative: Osmans, Voldemars
(86) International application number: PCT/RU2022/050313
(87) International publication number: WO 2023/085974

(57) **Abstract**

The invention relates to the field of smokers' requisites, and more particularly to electronic cigarettes, and can be used as a hardware and software system for helping to combat nicotine dependence. The aim of the invention is to improve and to increase the efficiency, in the proposed smart system for combating nicotine dependence, of a system for providing feedback to a user, specifically in the form of an indicator, and sending notifications and warnings and motivating the user by means of the electronic cigarette itself or by a software application. This aim is achieved in that in the claimed smart system for combating nicotine dependence, feedback is provided to the user by indicator light and vibratory modules, as well as by a piezoelectric noise emitter, which are built directly into an electronic cigarette, as well as by notifications and push notifications sent by a software application to a smartphone, tablet, laptop, computer, smart watch or bracelet, or chatbot messages, and by the possibility of deducting money from the user's credit card.

## Description

### FIELD OF THE INVENTION

The invention relates to the field of smoking accessories, namely electronic cigarettes, and can be used as a software and hardware complex to help combat nicotine dependence.

### BACKGROUND

From the publicly available state of the art, it is known that an electronic cigarette (e-cigarette) is an electronic device that creates a highly dispersed vapor (aerosol) intended for inhalation. It can be used both as an electronic nicotine delivery system (ENDS) and for inhaling flavored vapor (aerosol) without nicotine. Vapor is created by evaporation of a specially prepared liquid from the surface of a heating element.

The vapor visually resembles tobacco smoke. The main elements of the device are a mouthpiece, a lithium battery and an atomizer with a heating element, a cartridge or a tank with a special liquid filler (Wikipedia Free Encyclopedia// 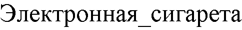 )[electronic resource] URL: https://ru.wikipedia.org/wiki/ 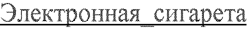 (accessed 10/22/2021).

Special liquid (E-liquid) is used as a consumable for vapor production in electronic cigarettes. To deliver nicotine to the user's body, the liquid is converted from a liquid aggregate state and turned into vapor via rapid heating. The user inhales this vapor, which includes nicotine and flavor. When exhaling, the vapor very much resembles the smoke of a regular cigarette. Liquid for electronic cigarettes consists of a base (a mixture of glycerin and propylene glycol), flavorings and nicotine. The user can choose the amount of nicotine and the ratio of glycerin and propylene glycol, which, in turn, allows the user to adapt more easily when switching from conventional cigarettes to electronic ones, and then gradually reduce the concentration of nicotine to abandon the bad habit.

Thus, the transition from smoking classic cigarettes with tobacco burning to electronic cigarettes allows the smoker to gradually reduce the degree of nicotine dependence and, in the future, strictly following the recommendations and gradually reducing the strength of the liquid, to abandon the bad habit (Russian BBC// 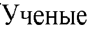 : 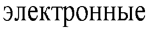 [electronic resource] URL:https://www.bbc.com/russian/science/2013/09/130909 e-cigarettes patches (accessed 10/22/2021).

E-cigarettes are used as an alternative to classic tobacco cigarettes to reduce and combat tobacco dependence and generally reduce harm to the smoker's health.

In this regard, to increase the effectiveness of combating nicotine dependence, manufacturers currently offer technical solutions for electronic cigarettes, which are part of an entire system to control the consumption of smoking mixture and reduce the degree of nicotine dependence. Such solutions (software and hardware) include an electronic cigarette and some combination of the following elements: the possibility of establishing a remote connection with an external device (smartphone, tablet, laptop or computer) using the built-in electronic cigarette and related microprocessor modules with Bluetooth (in rare cases Wi-Fi, radio frequency (RF), or NFC module), and/or USB connector for wired transmission and reception of data; a microprocessor or microcontroller; a software application on the external device with interface and various features for managing and monitoring nicotine consumption, and with feedback that can be transferred to a server or a cloud storage on the Internet that is associated, on the one end, with microprocessor and e-cigarette memory, on the other end, with a server or cloud service with available two-way transmission of data and commands between microprocessor via a Bluetooth module to a software application server, or cloud service; built-in electronic cigarette software; automatic collection, storage and analysis of consumption statistics (number, frequency and duration of puffs, total smoking time, amount of nicotine consumed, etc.) by a smoker in memory of the a database on a server or cloud; ability to predict and offer a personalized plan (program) to combat nicotine consumption, based on the collected statistics; feedback with sound and light indication on the electronic cigarette, or through notifications in the software application; establishment of restrictions on nicotine consumption by blocking the device, and overall management of the process through communication with other consumers via social network; communication with clinicians in real time through a software application, audio signal search function, and tools to block unauthorized access using a password, security key, fingerprint, and GPS, BDS or GIS positioning. The listed solutions are disclosed in Russian patent for invention No. 2655596 (priority date 05/23/2014), international registrations No. WO2015063126A1 (priority date 10/29/2013), WO2014195805A2 (priority date 05/20/2013), WO2018025217A1 (priority date 08/03/2016), WO2020140676A1 (priority date 01/04/2019), foreign patents for inventions CN110661918A (priority date 05.09.2019), CN1106681765A (priority date 12/24/2016), US2016219931A1 (priority date 30.01.2015), CN110419782A (priority date 07/18/2019), US8402976B2 (priority date 04/17/2008), CN11399937A (priority date 03/09/2020), US2014202477A1 (priority date 01/16/2014), CN110472401A (priority date 05.092019), KR20160108855A (priority date 09.03 2015), US2015366268A1 (priority date 12.02.2014), EP3275324A1 (priority date 07/25/2016).

As the closest analog to the claimed invention, a technical solution was chosen according to the Russian patent for invention No. 2680224 "APPLICATION DEVELOPMENT FOR NETWORK WITH ELECTRONIC CIGARETTE". In addition to the standard elements for an electronic cigarette, this solution also includes a controller, an air flow sensor, a storage device as part of an electronic cigarette, wireless communication with the device (PC, smartphone, tablet computer), ability to control smoking properties through a mobile application; ability to automatically turn off the power, reduce nicotine, activate the delay after a puff, reduce power, etc., in case of antismoking plan violation; setting restrictions on the number of cigarette uses, nicotine levels; using smartphone sensors, GPS, accelerometer, microphone or other smartphone elements; sending notifications to the user via email, text messages, instant messages or through a smartphone application; software: a smartphone application with an interface, a server, a database, two-way communication via smartphone; preventing unauthorized access by setting a password or a security key in a smartphone.

Common disadvantages of the above mentioned solutions include the limitations of the user feedback system, namely, indication and transmission of notifications, warnings, stimulation of the user via an electronic cigarette or a software application, while the function of monitoring and restricting nicotine consumption is limited; unclear implementation of audio warning system; and the notification system and the devices to which the notifications can be directed are limited to a smartphone, tablet, laptop, and computer.

### SUMMARY

The aim of the present invention is to eliminate the above-mentioned disadvantages: to increase the effectiveness of the proposed smart system for combating nicotine dependence with user feedback, namely, to improve indication, transmission of notifications, warnings, user stimulation via an electronic cigarette or a software application.

This aim is achieved by the fact that in the claimed smart system for combating nicotine addiction, user feedback is implemented via light indication and vibration modules, a piezoelectric sound emitter built directly into an electronic cigarette, and by sending push notifications to a smartphone, tablet, laptop, computer, smart watches, bracelets, chats; and the claimed device is also able to withdraw funds from a user's credit card.

The technical result of the claimed solution "SMART SYSTEM FOR COMBATING NICOTINE ADDICTION" is as follows.

The proposed smart system for combating nicotine dependence has improved the user feedback system, which is implemented simultaneously using light indication, vibration and piezoelectric sound modules built directly into the electronic cigarette, and by sending notifications to a smartphone, tablet, laptop, computer, smartwatch, smart bracelet, messages to chats; and the system is able to withdraw funds from the user's credit card. As a result of this user feedback system implementation, the impact on user behavior becomes more diverse and effective, which generally increases the effectiveness of controlling and limiting user consumption of the smoking mixture; allows faster achievement of the goals of nicotine consumption control, and potentially achieves termination of nicotine dependence.

Also, the claimed invention expands the arsenal of technical solutions for electronic cigarettes as part of a software and hardware complex to help combat nicotine dependence.

The technical result is achieved due to the fact that the smart system for combating nicotine dependence is a software and hardware complex consisting of an electronic cigarette containing a cartridge that includes a mouthpiece, a container with liquid, a filter, a heater, a wick for supplying smoking liquid, contacts for connecting and supplying power to the heater, cartridge identification elements, and an electronic cigarette case that includes a frame, an outer case, a power supply module, a two-way wireless communication board, a control board with pressure measurement modules, a light indication, an electronic control and work control module for the device, a storage device with embedded software, an LED, a USB connector on an external user device, a software application on an external device with an interface and the ability to send notifications and messages to the user, and a virtual analytical server including a database, a software interface and a computer analytical service, characterized in that the electronic cigarette cartridge additionally contains a piezoelectric sound emitter; vibration motor and audio alarm modules are additionally installed on the control board; a smartphone, tablet, laptop, computer, smartwatch or smart bracelet act as external devices; and the software application is designed with the ability to send additional push notifications to an external device and send messages to chats, as well as the ability to link the software application to the user's credit card.

### DRAWINGS

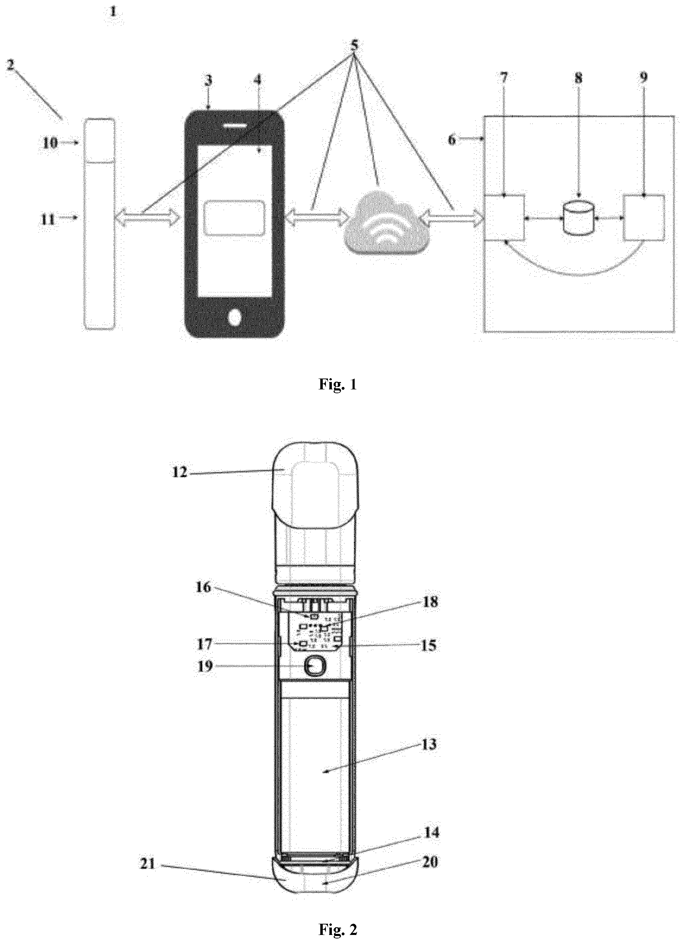

The claimed technical solution is illustrated by schematic images of a smart system for combating nicotine dependence (Figure 1) and an electronic cigarette as part of this system (Figure 2), explaining the implementation of the proposed invention within the claims, where 1 is a software and hardware complex with all its elements, 2 is an electronic cigarette, 3 is an example of an external device with a software application installed on it with an interface 4, 5 are two-way wireless communication between an electronic cigarette, an external device and a virtual analytical server, 6 is a virtual analytical server, 7 is a software interface, 8 is a database, 9 is a computer analytical service, 10 is a cartridge, 11 is an electronic cigarette case, 12 is a mouthpiece, 13 is a power supply module, 14 is a two-way wireless communication board, 15 is a control board, 16 is a pressure measurement module, 17 is a light indication and audio alarm module, 18 is a vibration motor module, 19 is a LED, and 20 is a USB connector.

### DETAILED DESCRIPTION OF THE INVENTION

To achieve the desired technical result, the invention can be carried out in the following preferred way, without excluding other methods of implementation within the utility model claims.

The proposed smart system for combating nicotine dependence is a software and hardware complex (1), which is designed primarily to ensure the process of smoking (vaping) by the user of an electronic cigarette (2). The operation of all elements of this software and hardware complex (1) allows you to automatically collect statistics on the user's consumption of smoking liquid, store it, analyze it, build a portrait (characteristics) of the smoker's consumption, develop an individual plan and recommendations to combat nicotine dependence, monitor the implementation of the plan and user behavior during smoking, and adjust the plan if necessary.

To achieve this technical result, the smart system for combating nicotine dependence works as follows.

A user purchases an electronic cigarette (2), installs on an external device (smartphone, tablet, laptop, computer) (3) a special software application with an interface (4) that connects the external device (2) with an electronic cigarette (2) via wireless communication (5).

The user begins the process of smoking (vaping) a liquid located in a container (not shown in the diagrams) inside the cartridge (10). The cartridge (10) is connected to the case of an electronic cigarette (11) by means of contacts (not shown in the diagrams), and also contains a container with smoking liquid, a filter, a heater, a wick for supplying smoking liquid, contacts for supplying power to the heater, and cartridge identification elements (not shown in the diagrams). The electronic cigarette case (11) also contains a USB connector (20) for charging the power module (13), frame, outer case, electronic control and work control module of the device, and a storage device with embedded software, which are not shown in the diagrams.

Next, the user consumes the vaping liquid for several days. During this time, the smart system collects, stores and analyzes statistics on the user's consumption of smoking liquid, and then, on this basis, a plan and recommendations for combating nicotine dependence are developed. These processes are carried out as follows:
During smoking, the pressure measurement module (16) located on the control board (15) receives statistical information about the puff (speed and volume, puff depth, sharpness), and in the storage device (not shown in the diagrams) on the control board (15), the duration and frequency of puffs, data on the amount of consumed nicotine and evaporated smoking liquid are also recorded. The general control of all elements of the proposed invention is carried out by embedded software in a storage device (not shown in the diagrams).

Next, the statistics collected on the control board (15) are transmitted via the wireless communication board (14) to an external device (3). The wireless communication (5) between the wireless communication board (14) and an external device (3) is two-way, preferably using Bluetooth technology, but it can also be implemented using Wi-Fi, radio frequency (RF) or NFC technologies. Two-way wireless communication (5) is carried out with an external user device (3) (smartphone, tablet, laptop, computer, smartwatch, smart bracelet) and allows to automatically transmit (without user participation) statistics collected by the control board elements (15) on the number of puffs from an electronic cigarette (2), their duration, the characteristics of puffs, the amount of nicotine consumed, and the amount of evaporated smoking liquid to a software application installed on an external device (3) with an interface (4) and then to a virtual analytical server (6) for mathematical calculations, which allows you to build a portrait (characteristics) of a smoker.

The software application (4) provides two-way communication, on the one end, through the wireless communication board (14) with the control board (15) of the electronic cigarette (2), and on the other end, with a virtual analytical server (6), which receives statistics on the consumption of smoking liquid and generates control functions in accordance with the control algorithms. Thus, the software application (4) receives all the parameters of the smoking liquid consumption through the wireless communication board (14) and the modules installed on the control board (15) of the electronic cigarette, collects and transmits them to the virtual analytical server (6). In addition, the software application (4) in real time via an interface on an external device (3) informs the user about the work of e-cigarette (2), and shows related situational messages about consumption indicators and demonstrates a plan and recommendations for reducing nicotine consumption, sends motivating messages, allows to control the operation of the electronic cigarette (2), including optimizing the puff properties on the electronic cigarette (2), depending on the established daily consumption rates of smoking liquid, the installed cartridge (10), and the user's smoking manner, and updates the software (not shown in the diagrams) built into the electronic cigarette.

The virtual analytical server (6) includes a software interface (7), a database (8) of statistics on user consumption of smoking liquid, and a computer analytical service (9), in which the analysis of statistics on user consumption of smoking liquid is carried out via mathematical calculations; behavioral analysis of the user is performed, after that recommendations, tips, and plans for reducing nicotine dependence are automatically developed based on machine learning, taking into account the actual incoming data from the user.

Thus, in a smart system for combating nicotine dependence, several software and hardware elements are used, which allow automatic collection of all statistics on the user's consumption of smoking liquid. It is achieved due to (15) which is a wireless communication board, (14) which is a software application on an external device, (4) is a virtual analytical server (6); the statistics is then stored, analyzed and used to create a user's portrait (characteristics) to develop an individualized plan to combat nicotine dependence and to adjust that plan if necessary.

Direct monitoring of the implementation of the plan developed by the computer analytical service (9) and the recommendation to combat nicotine dependence is carried out by the proposed smart system using light indication modules (17), vibration motor (18), audio alarm (17) built into the control board (15) of the electronic cigarette (2), a LED (19), and a piezoelectric sound emitter (not shown in the diagrams).

The LED (19) is preferably realized as an RGB-LED. The audio alarm module (17), through a piezoelectric sound emitter (not shown in the diagrams), sends signals to the user to control the consumption of the smoking mixture, and also allows to localize the electronic cigarette in case of loss. The vibration motor module (18) produces a tactile effect on the user's fingers and lips, and also helps to locate the electronic cigarette (2). Thus, in case of non-fulfillment of the plan or, conversely, achievement of certain goals, the smart system automatically transmits light, sound and tactile signals to the user. That is, the user is informed and warned in a variety of ways about violations or achievements, which leads to correcting of behavior.

In addition, notifications and messages to the user are sent to the display of the external device (3) through the software application installed on it (4), not only directly on the application interface (4), but also in the form of push notifications to an external device (3), e. g., to a smartphone, smart watch, smart bracelet, and chats. Thus, the user is informed that today he or she has consumed more nicotine than planned, that he or she has consumed a lot in a short time, and that it is necessary to correct behavior. The software application (4) also sends messages, gamifying the process.

It is also possible to link the user's credit card data (not shown in the diagrams) to the software application (4) to withdraw funds as a penalty and transfer them to a special account in case the user violates the requirements of the plan.

Thus, the claimed invention implies a complete user feedback system, in which the impact on user behavior becomes more diverse and effective, which generally increases the effectiveness of controlling and limiting user consumption of the smoking mixture; allows faster achievement of the goals of nicotine consumption control, and potentially achieves termination of nicotine dependence.

The novelty and inventive step of the presented invention consists in the fact that the totality of its essential features provides a more diverse and effective impact of the smart system on user behavior when using an electronic cigarette, which generally increases the effectiveness of controlling and limiting user consumption of the smoking mixture; allows faster achievement of the goals of nicotine consumption control, and potentially achieves termination of nicotine dependence.

The applicant is currently carrying out the production of the proposed invention.

## Claims

1. A smart system for combating nicotine dependence comprising
a software and hardware complex consisting of an electronic cigarette containing a cartridge that includes a mouthpiece, a container with liquid, a filter, a heater, a wick for supplying smoking liquid, contacts for connecting and supplying power to the heater, cartridge identification elements, and an electronic cigarette case that includes a frame, an outer case, a power supply module, a two-way wireless communication board, a control board with pressure measurement modules, a light indication, an electronic control and work control module for the device, a storage device with embedded software, a LED,
an external user device containing a USB connector, a software application on the external device with an interface and the ability to send notifications and messages to the user,
a virtual analytical server including a database, a software interface and a computer analytical service, **characterized in that**
the electronic cigarette cartridge additionally contains a piezoelectric sound emitter;
a vibration motor and an audio alarm modules additionally installed on the control board;
wherein the external user device chose from a smartphone, tablet, laptop, computer, smartwatch or smart bracelet;
and the software application is designed with the ability to send additional push notifications to the external device and send messages to chats, as well as the ability to link the software application to the user's credit card.
